# EUROPEAN PATENT SPECIFICATION

(11) **EP 1 837 051 B1**
(45) Date of publication and mention of the grant of the patent: **13.06.2012**
(21) Application number: 05771731.6
(22) Date of filing: 02.08.2005
(51) Int. Cl.: A61N 7/02

(54) **HIGH INTENSITY FOCUSED ULTRASOUND THERAPEUTIC APPARATUS**
FOKUSSIERTES THERAPEUTISCHES HOCHINTENSITÄTSULTRASCHALLGERÄT
APPAREIL THERAPEUTIQUE A ULTRASONS FOCALISES A HAUTE INTENSITE

(30) Priority: 10.01.2005 CN 200510000351
(43) Date of publication of application: 26.09.2007
(73) Proprietor: CHONGQING HAIFU(HIFU)TECHNOLOGY CO., LTD, Chongqing 401121 (CN)
(72) Inventor: CHEN, Wenzhi, Yubei District, Chongqing 401121 (CN); WANG, Zhibiao, Yubei District, Chongqing 401121 (CN); WANG, Zhilong, Yubei District, Chongqing 401121 (CN); LIN, Tao, Yubei District, Chongqing 401121 (CN); ZHAO, Chunliang, Yubei District, Chongqing 401121 (CN)
(74) Representative: Cohausz & Florack
(86) International application number: PCT/CN2005/001175
(87) International publication number: WO 2006/072196

(56) References cited:
- WO-A2-01/82777
- CN-Y- 2 551 208
- DE-A1- 19 506 363
- US-A- 4 932 414
- US-A1- 2003 153 961
- US-B1- 6 685 639

## Description

### FIELD OF THE INVENTION

This invention relates to an apparatus for high-intensity focused ultrasound therapy, particularly it relates to an apparatus for switching the emissions of the therapeutic ultrasound beams according to gray-scale changes of ultrasound images.

### BACKGROUND OF THE INVENTION

Medical researchers have discovered that the heat-resistance of cancer cells is poorer than that of normal cells. When the temperature is above 42.5 degrees centigrade, the cancer cells die within 30 minutes while the normal cells are injured slightly and the injury is reversible. According to this characteristic of cancer cells, a high-intensity focused ultrasound tumor therapeutic system makes use of ultrasound waves as energy source and their penetrability and focusing, and delivers the ultrasound waves with a relatively low average intensity level emitting from the applicator coupled by medium through skin into the tumor tissue within human body. These ultrasound beams within tissue are focused at a spatial focal spot and a focal region (with a size of Φ 3 mm X 8mm) with an average acoustic intensity level of over 1000W/m² is formed. The temperature at the focal region instantaneously (0.1 to 5 seconds) has a sharp rise (beyond 70 degrees centigrade). By this thermal effect together with its cavitation (i.e. the high-intensity ultrasound may produce fog-like bubbles in liquid and the mechanical forming and disruption of the bubbles can produce extremely high temperature and pressure, which can destroy the tissue structure greatly) and mechanical action, the disruption of the tissue at the focal region can be achieved. The cancer cells can be exposed by the focal region through the scanning way from point to line, then from line to slice and then from slice to solid and finally the whole tumor tissue is cauterized and the therapeutic purposes is realized.

With the development of ultrasound therapy, great improvements on checking and treating tumors by ultrasound therapy associated with imaging devices, such as B-mode ultrasound have been achieved. The B-mode ultrasound imaging apparatus used in the existing ultrasound therapy equipments is mainly used to locate and observe tumor prior to ultrasound therapy. By use of MRI or CT checking for some time after treatment, the existing equipments determine if the needed therapeutic dosage has been achieved at the target area after treatment, if the coagulative necrosis of target tissue is caused, and evaluate the therapeutic effects after treatment. In this way, a lot of disadvantages exist because the therapeutic effects cannot be observed in time and the therapeutic dose is controlled only by experiences. If the dose is insufficient, the diseased tissue cannot be destroyed effectively and the therapeutic effects cannot be achieved. If the dose exceeds the needed dose, the normal tissue may be destroyed and there exists a big safety problem. Therefore, the real-time evaluation on the therapeutic effects during treatment becomes very necessary.

### SUMMARY OF THE INVENTION

In order to solve the problems as above-mentioned, the apparatus for high-intensity focused ultrasound therapy of the present invention comprises a ultrasound transducer to emit high-intensity focused ultrasound to the target area to be treated, an image acquisition device for locating the target area to be treated and acquiring the images of the target area, a driving unit connected to said ultrasound transducer for driving said ultrasound transducer to emit ultrasound waves to the target area to be treated and mechanical drive means connected to said ultrasound transducer for mechanically driving said ultrasound transducer and image acquisition device together so as to scan the target area to be treated. Wherein said apparatus for high-intensity focused ultrasound therapy further comprises a signal processing unit connected to image acquisition device for receiving and saving the original images of the target area acquired by image acquisition device prior to treatment and for receiving and saving the images of the target area acquired by image acquisition device in a certain time during high-intensity focused ultrasound (HIFU) treatment and for evaluating the therapeutic effects according to the images of the target area before and after treatment, and a control unit respectively connected to signal processing unit, driving unit and mechanical driving means. When the signal processing unit judges that the therapeutic requirements have been met, the control unit controls the driving unit and mechanical driving means to stop the working of ultrasound transducer and when the signal processing unit judges that the therapeutic requirements have not been met, the control unit controls the driving unit and mechanical driving means to make the ultrasound transducer continue to work.

Additionally, a method for high-intensity focused ultrasound therapy comprises the following steps: acquiring original images, i.e. receiving and acquiring the original images of the target area to be treated prior to treatment; making a treatment, i.e. emitting high-intensity focused ultrasound and applying it to the target area; evaluating the therapeutic effects, i.e. acquiring and saving the images of the target area in a certain time during HIFU treatment and evaluating the therapeutic effects according to the images of the target area before and after treatment. When the signal processing unit judges that the therapeutic requirements have been met, the treatment on the target area is stopped and when the signal processing unit judges that the therapeutic requirements have not been met, the treatment on the target area is continued.

According to the apparatus for high-intensity focused ultrasound therapy of the present invention, the therapeutic effects of the target area can be evaluated in real-time during treatment and the treatment on the target area is decided to continue or stop according to these evaluations. Accordingly the treatment dosage can be controlled accurately and the optimal therapeutic effects can be achieved.

### BRIEF DESCRIPTION OF THE FIGURES

Fig.1 is a block diagram of an apparatus according to the invention.
Fig. 2 is a working flow diagram according to the invention.
Fig. 3 is B-mode ultrasound image of a tissue prior to treatment.
Fig. 4 is B-mode ultrasound image of a tissue after treatment.
Fig. 5 is B-mode ultrasound image of a tissue prior to treatment.
Fig. 6 is B-mode ultrasound image of a tissue after treatment.

### DETAILED DESCRIPTION OF PREFERRED EMBODIMENTS

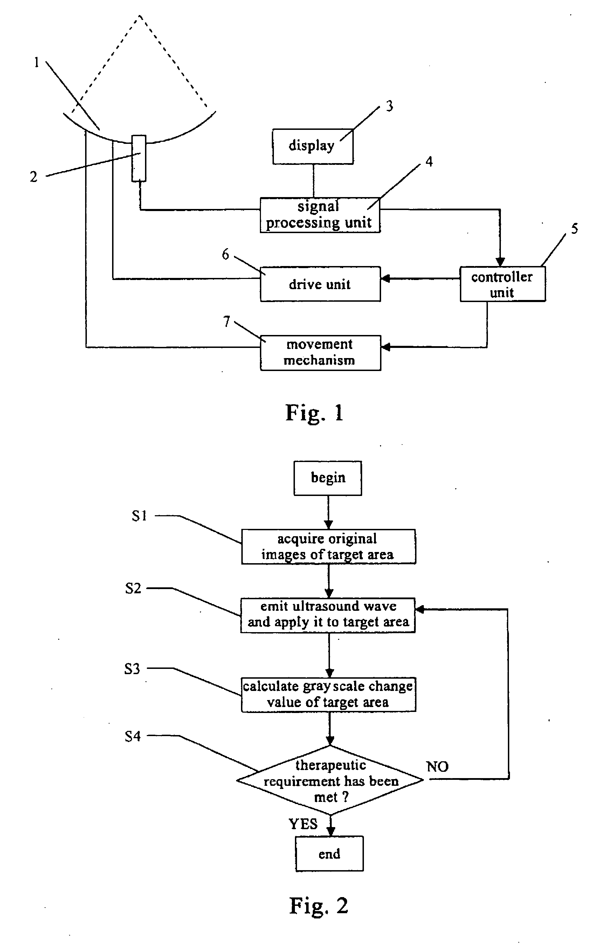

The structure of the apparatus according to the present invention is explained according to Fig.1. As shown in Fig.1, the apparatus for high-intensity focused ultrasound therapy of the present invention usually comprises ultrasound transducer 1, image acquisition device 2, driving unit 6 and mechanical drive means 7. Besides, in order to realize the purposes of the present invention, it further comprises signal processing unit 4 and control unit 5. Wherein, the ultrasound transducer 1 emits high-intensity focused ultrasound to the target area to be treated and the image acquisition device 2 is installed on the ultrasound transducer 1 and is used to locate the tumor and acquiring the images of the target area and the existing imaging devices can be used and B-mode ultrasound imaging apparatus as an example is described in this invention. The signal processing unit 4 is connected to the image acquisition device 2 for receiving and saving the images of the target area acquired by image acquisition device 2. The gray scales of the current images of the target area can be worked out and evaluated on the basis of the original image data before treatment. The display 3 is connected to the signal processing unit 4 for displaying the images outputted from the signal processing unit 4. The driving unit 6 connected to the ultrasound transducer 1 drives the ultrasound transducer 1 to emit ultrasound waves to the target area to be treated under control of the control unit 5. The mechanical drive means 7 connected to the ultrasound transducer 1 mechanically move the ultrasound transducer 1 and the B-mode ultrasound imaging apparatus 2 together under control of the control unit 5. The control unit 5 connected respectively to signal processing unit 4, driving unit 6 and mechanical driving means 7 receives the computation and evaluation of gray scale changes of the images of the target area outputted from the signal processing unit 4 and controls the working of the driving unit 6 and mechanical driving means 7 according to the computing and evaluating results.

The working flow diagram of the apparatus for the high-intensity focused ultrasound therapy of the present invention is explained according to Fig.2. As shown in Fig.2, the step S1 is entered after starting treatment. In the step S1, B-mode ultrasound imaging apparatus 2 acquires the original images of the target area to be treated and transfers them to the signal processing unit 4. The signal processing unit 4 works out the primary gray scales of these images and saves these primary gray scales for later computation and evaluation.

In the step S2, the control unit 5 controls the driving unit 6 and mechanical driving means 7 and starts the ultrasound transducer 1 to emit the high-intensity focused ultrasound to scan the target area and expose the tumor cells in the target area by the focused ultrasound.

In a certain time, for example, after at least one scanning, the step S3 is entered, B-mode ultrasound imaging apparatus 2 acquires the images of the target area to be treated again and transfers them to the signal processing unit 4. The signal processing unit 4 works out the gray scales of these images after treatment and then works out the gray scale changes of these images after treatment according to the saved primary gray scales.

Then, the step S4 is entered. In this step, the signal processing unit 4 determines if the gray scale changes exceed a specified value. If the gray scale changes exceed the specified value, it means that acoustic therapy dose on the target area has been reached and at this time the emission of high-intensity focused ultrasound can be stopped. On the contrary, if the gray scale changes do not exceed the specified value, it means that acoustic therapy dose on the target area has not been reached and at this time the emission of high-intensity focused ultrasound for treatment shall be continued. With mass experiments on animal samples, this inventor has discovered that the gray scale changes of ultrasound images of the target area before and after treatment is related to not only the acoustic power and exposure time, but also the exposure depth, tissue structure, function status of tissue and treatment modes. But, if only the gray scale changes exceed 5 to 10 gray scales, the coagulative necrosis of the tumor tissue in the target area happens. In the present invention, the gray scale values are averagely distributed in the range of [0∼255]. The value 0 represents full black, 255 represents full white.

The calculating method for gray scales and gray scale changes is using the computer to acquire the images from the monitoring device B-mode scanner in a special time, for example the static images before and after treatment. The computer system automatically records the position, energy and equipment parameters when the images are acquired. By use of computer graphics arithmetic, such as difference operation and convolution operation, the gray scales changes at the specified place can be compared. The comparing area may be a dot, line (straight line, curve, treatment track and etc.), slice (rectangle, manual drawing) and etc. According to the positions of treatment tracks on the images before and after treatment, the gray scale characteristic values in a certain area are calculated, for example, total gray scale value, average gray scale, gray scale weighted value (summation, averaging and etc.), gray scale of area center and etc.. By use of difference operation and convolution operation, the gray scale characteristic values before and after treatment are compared.

In the step S4, if the gray scale changes of the images of the target area exceed a specified value, the signal processing unit 4 sends a command of stop to the control unit 5. With the command of stop, the control unit 5 controls the driving unit 6 and the mechanical driving means 7 to stop ultrasound transducer 1 to emit high-intensity focused ultrasound and to scan, i.e. to stop the treatment.

In the step S4, if the gray scale changes of the images of the target area do not exceed a specified value, the signal processing unit 4 sends a command of continuing treatment to the control unit 5 and the step S2 is returned. In the step S2, with the command of continuing treatment, the control unit 5 controls the driving unit 6 and the mechanical driving means 7 to make ultrasound transducer 1 continue to emit high-intensity focused ultrasound and to scan, i.e. to continue the treatment.

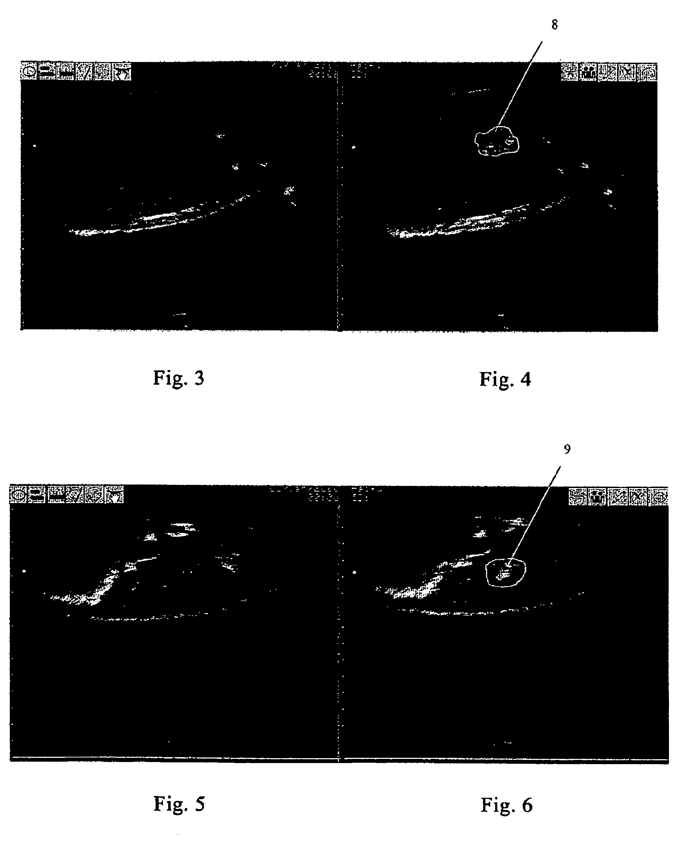

Fig. 3 and Fig. 4 are the B-mode ultrasound images for the same liver tissue before and after treatment. Fig. 3 is the image before treatment and Fig. 4 is the image after high-intensity focused ultrasound therapy. From the image shown in Fig. 4, we can see the gray scales changes at the focal point in the target area and we can find this part of liver tissue has become coagulative necrosis after it is anatomized and the therapeutic requirements have been met. This proves that this method used to evaluate the therapeutic effects is effective.

Fig. 5 and Fig. 6 are the B-mode ultrasound images for another liver tissue before and after treatment. Fig. 5 is the image before treatment and Fig. 6 is the image after high-intensity focused ultrasound therapy. The results and phenomenon are the same as that of Fig. 3 and Fig. 4. Here they are not repeated.

As above said, through calculating the gray scale changes of images of the target area, the therapeutic effects can be evaluated in real-time during treatment and the dose of high-intensity focused ultrasound can be adjusted according to the evaluating results. It can be avoided that the tumor tissue may not be killed effectively due to insufficient dose or the normal tissue may be destroyed due to excessive dose.

The present invention may be embodied in other specific forms without departing from its scope. The described embodiments are the specific embodiments of the present invention. These embodiments are to be considered in all respects only as illustrative, and not restrictive.

## Claims

1. An apparatus for high-intensity focused ultrasound therapy, comprising:
an ultrasound transducer to emit high-intensity focused ultrasound to a target area to be treated;
- an image acquisition device for locating said target area to be treated and acquiring images of said target area;
- a driving unit connected to said ultrasound transducer for driving said ultrasound transducer to emit ultrasound waves to said target area to be treated;
- mechanical drive means connected to said ultrasound transducer for mechanically driving said ultrasound transducer and said image acquisition device together so as to scan said target area to be treated;
- a signal processing unit connected to said image acquisition device for receiving and saving original images of said target area acquired by said image acquisition device prior to treatment and for receiving and saving images of said target area acquired by said image acquisition device in a certain time during high-intensity focused ultrasound treatment and for evaluating therapeutic effects according to said images of said target area before and after treatment; and
- a control unit respectively connected to said signal processing unit, said driving unit and said mechanical driving means, said control unit, when said signal processing unit judges that therapeutic requirements have been met, controlling said driving unit and said mechanical driving means to stop working of said ultrasound transducer, and said control unit, when said signal processing unit judges that said therapeutic requirements have not been met, controlling said driving unit and said mechanical driving means to make said ultrasound transducer continue to work, wherein
- said signal processing unit is adapted to calculate respectively a gray scale of said image of said target area before treatment in a gray scale range and a gray scale of said image of said target area after treatment in said gray scale range, and then calculate gray scale changes between said images of said target area before and after treatment,
**characterized in that**
- said gray scale range has 256 gray scales, and
- said signal processing unit judges that said therapeutic requirement has been met if said gray scale changes exceed a value of 5 to 10 gray scales.

2. The apparatus for high-intensity focused ultrasound therapy according to claim 1, wherein said certain time is the time after said ultrasound transducer has scanned said target area to be treated at least for one time.

## Patentansprüche

1. Vorrichtung zur fokussierten Hochintensitäts-Ultraschalltherapie, umfassend:
- einen Ultraschallwandler zum Emittieren von fokussiertem Hochintensitäts-Ultraschall auf einen zu behandelnden Zielbereich,
- ein Büderfassungsgerät zum Lokalisieren des zu behandelnden Zielbereichs und zum Erfassen von Bildern der Zielbereich,
- eine mit dem genannten Ultraschallwandler verbundene Ansteuereinheit zum Ansteuern des Ultraschallwandlers zum Emittieren von Ultraschallwellen auf den zu behandelnden Zielbereich ,
- eine mit dem genannten Ultraschallwandler verbundene mechanische Antriebseinrichtung zum gemeinsamen mechanischen Antreiben des Hochintensitäts-Ultraschallwandlers und des Bilderfassungsgeräts, um den zu behandelnden Zielbereich abzutasten,
- eine mit dem Bilderfassungsgerät verbundene Signalverarbeitungseinheit zum Empfangen und Abspeichern von Originalbildern des Zielbereichs, die von dem Bilderfassungsgerät vor der Behandlung erfasst werden, und zum Empfangen und Abspeichern von Bildern des Zielbereichs, die von dem Bilderfassungsgerät zu einem vorgegebenen Zeitpunkt während der Behandlung mit fokussiertem Hochintensitäts-Ultraschall erfasst werden, und zum Bewerten therapeutischer Wirkungen entsprechend den Bildern des Zielbereichs vor und nach der Behandlung, und
- eine Steuereinheit, die mit der Signalverarbeitungseinheit, der Ansteuereinheit und der mechanischen Antriebseinrichtung verbunden ist, wobei die Steuereinheit, wenn die Signalverarbeitungseinheit urteilt, dass die therapeutischen Anforderungen erfüllt sind, die Ansteuereinheit und die mechanische Antriebseinheit derart ansteuert, dass der Betrieb des Ultraschallwandlers beendet wird, und wobei die Steuereinheit, wenn die Signalverarbeitungseinheit urteilt, dass die therapeutischen Anforderungen nicht erfüllt sind, die Ansteuereinheit und die mechanische Antriebseinheit derart ansteuert, dass ein fortgesetzter Betrieb des Ultraschallwandlers bewirkt wird, wobei
- die Signalverarbeitungseinheit dazu ausgebildet ist, eine Graustufe des Bilds des Zielbereichs vor der Behandlung in einem Graustufenbereich und eine Graustufe des Bilds des Zielbereichs nach der Behandlung in dem Graustufenbereich zu berechnen und anschließend Graustufenänderungen zwischen den Bildern des Zielbereichs vor und nach der Behandlung zu berechnen ,
**dadurch gekennzeichnet, dass**
- der Graustufenbereich 256 Graustufen hat und
- die Signalverarbeitungseinheit urteilt, dass die therapeutische Anforderung erfüllt ist, wenn die Graustufenänderungen einen Wert von 5 bis 10 Graustufen übersteigen.

2. Vorrichtung zur fokussierten Hochintensitäts-Ultraschalltherapie nach Anspruch 1, wobei der vorgegebene Zeitpunkt der Zeitpunkt ist, nachdem der Ultraschallwandler den zu behandelnden Zielbereich wenigstens einmal abgetastet hat.

## Revendications

1. Appareil destiné à une thérapie par ultrasons à haute intensité focalisés comprenant :
- un transducteur ultrasons destiné à émettre des ultrasons focalisés à haute intensité vers une zone cible à traiter,
- un dispositif d'acquisition d'images destiné à localiser ladite zone cible à traiter et à acquérir des images de ladite zone cible,
- une unité de commande raccordée audit transducteur ultrasons de façon à commander ledit transducteur ultrasons pour émettre des ondes ultrasons vers ladite zone cible à traiter,
- un moyen d'entraînement mécanique raccordé audit transducteur ultrasons pour entraîner mécaniquement ledit transducteur ultrasons et ledit dispositif d'acquisition d'images conjointement afin de scanner ladite zone cible à traiter,
- une unité de traitement de signaux raccordée audit dispositif d'acquisition d'images pour recevoir et enregistrer des images originales de ladite zone cible acquises par ledit dispositif d'acquisition d'images avant le traitement et pour recevoir et enregistrer des images de ladite zone cible acquises par ledit dispositif d'acquisition d'images à un certain temps au cours du traitement par ultrasons à haute intensité focalisés et pour évaluer des effets thérapeutiques en fonction desdites images de ladite zone cible avant et après traitement, et
- une unité de contrôle respectivement raccordée à ladite unité de traitement de signaux, à ladite unité de commande et audit moyen d'entraînement mécanique, ladite unité de contrôle, lorsque ladite unité de traitement de signaux estime que des exigences thérapeutiques ont été satisfaites, commandant ladite unité de commande et ledit moyen d'entraînement mécanique de façon à arrêter le fonctionnement dudit transducteur ultrasons et, ladite unité de contrôle, lorsque ladite unité de traitement de signaux estime que lesdites exigences thérapeutiques n'ont pas été satisfaites, commandant ladite unité de commande et ledit moyen d'entraînement mécanique de façon à faire ledit transducteur à ultrasons continuer à opérer, où
- ladite unité de traitement de signaux est adaptée de façon à calculer un niveau de gris de ladite image de ladite zone cible avant traitement dans une gamme de niveaux de gris et un niveau de gris de ladite image de ladite zone cible après traitement dans ladite gamme de niveaux de gris et à calculer ensuite des changements dans le niveau de gris entre lesdites images de ladite zone cible avant et après traitement,
**caractérisé en ce que**
- ladite gamme de niveaux de gris compte 256 niveaux de gris, et
- ladite unité de traitement du signal estime que ladite exigence thérapeutique a été satisfaite si les changements dans le niveau de gris dépassent une valeur de 5 à 10 niveaux de gris.

2. Appareil destiné à une thérapie par ultrasons à haute intensité focalisés selon la Revendication 1, où ledit certain temps est le temps après que ledit transducteur ultrasons ait scanné ladite zone cible à traiter au moins une fois.
